## Europäisches Patentamt

## European Patent Office

## Office européen des brevets

(19)

(11) Veröffentlichungsnummer: **0 033 543**
**B1**

(12) **EUROPÄISCHE PATENTSCHRIFT**

(45) Veröffentlichungstag der Patentschrift:
02.01.85

(51) Int. Cl.³: **G 01 N 33/72**

(21) Anmeldenummer: 81100762.4

(22) Anmeldetag: 03.02.81

(54) Verfahren zur Bestimmung einer speziellen Hämoglobinspezies und Verwendung dieses Verfahrens zur Bestimmung von Diabetes.

(30) Priorität: 04.02.80  CH 868/80

(43) Veröffentlichungstag der Anmeldung:
12.08.81 Patentblatt 81/32

(45) Bekanntmachung des Hinweises auf die Patenterteilung:
02.01.85 Patentblatt 85/1

(84) Benannte Vertragsstaaten:
CH DE FR GB IT LI NL

(56) Entgegenhaltungen:
DE - A - 2 820 310
DE - A - 2 950 457
DE - A - 3 016 555
GB - A - 2 024 829
US - A - 4 108 603

DIABETES, Band 28, April 1979, New York, K.H. GABBAY et al. "Glycosylated Hemoglobins: Increased Glycosylation of Hemoglobin A in Diabetic Patients", Seiten 337 bis 340

(73) Patentinhaber: F. HOFFMANN-LA ROCHE & CO. Aktiengesellschaft, CH-4002 Basel (CH)

(72) Erfinder: Glatthaar, Beat Erich, Dr., St. Johannsvorstadt 62, CH-4056 Basel (CH)

(74) Vertreter: Lederer, Franz, Dr. et al, Patent Attorneys Dr. Franz Lederer Reiner F. Meyer 22 Lucile-Grahn-Strasse, D-8000 Munich 80 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Es ist bekannt, daß die Erythrozyten von Erwachsenen neben der meistvertretenen Hämoglobinspezies A (ca. 90%) und $A_2$ (ca. 2,5%) sowie kleinen Mengen der Spezies F (ca. 0,5%) noch weitere Hämoglobinspezies enthalten. Diese Hämoglobinspezies machen ca. 5—8% des Totalhämoglobins aus. Von diesen Hämoglobinspezies ist das $A_{1c}$ mengenmäßig das bedeutendste, da es 4—6% des gesamten Hämoglobins ausmacht. Es ist bekannt, das Diabetiker erhöhte Werte der Hämoglobinspezies $A_{1c}$ aufweisen [Rahbar et al. Biochem. Biophys. Res. Commun. 36, 838—843 (1969)]. Es ist weiterhin bekannt, daß die $HbA_{1b}$ Werte nicht die aktuellen Blutzuckerwerte wiederspiegeln, sondern als Integral der vorausgegangenen vier Monate zu werten sind, weshalb die Bestimmung von $HbA_{1b}$ von besonderem diagnostischen Wert ist.

$HbA_{1c}$ kann durch Kationenaustauschchromatographie von Hämolysaten von roten Blutzellen bestimmt werden [Trivelli L. A. et al. N. Engl. J. Med. 824, 353—357 (1971)], doch ist diese Methode zu aufwendig, um für Reihenuntersuchungen verwendet werden zu können. Es ist weiterhin bekannt, die Hämoglobinspezies $A_{1c}$ dadurch zu bestimmen, daß man Hämolysate von roten Blutzellen in saurer Lösung inkubiert, daß man die Proteine ausfällt, nach dem Abtrennen der Proteine eine Farbreaktion durchführt und anschließend nach Inkubieren eine photometrische Bestimmung vornimmt. Der erhaltene Werte wird danach in an sich bekannter Weise mit einem Standard verglichen. [Gabbay, K. H. et al. Diabetes Vol. 28 (1979)].

Im Rahmen der vorliegenden Erfindung wurde nun gefunden, daß man die vorerwähnte photometrische Bestimmungsmethode für die Hämoglobinspezies $A_{1c}$ wesentlich vereinfachen kann, indem man einerseits die Bestimmung nicht mit einem Hämolysat von roten Blutzellen durchführt, sondern in Vollblut und indem man andrerseits das Ausfällen der Proteine und die Farbreaktion simultan durchführt.

Die vorliegende Erfindung betrifft demnach ein Verfahren zur Bestimmung der Hämoglobinspezies $A_{1c}$ nach der photometrischen Methode, welche dadurch gekennzeichnet ist, daß man eine Vollblutprobe bei erhöhter Temperatur in saurer Lösung inkubiert, anschließend simultan die Proteine ausfällt und die Farbreaktion durchführt und daß man nach weiteren Inkubieren und Abtrennen der denaturierten Proteine eine photometrische Bestimmung durchführt und den erhaltenen Wert in an sich bekannter Weise mit einem Standard vergleicht.

Die erste Inkubation der Vollblutprobe erfolgt bevorzugt bei einer Temperatur von 95—105°C. Es wird bevorzugt während ein bis fünf Stunden, besonders bevorzugt während fünf Stunden inkubiert.

Als saure Lösung dient zweckmäßigerweise eine 0,2-I N Oxal-, Schwefel- oder Salzsäure-Lösung, bevorzugt ist eine 0,4 N Oxalsäure-Lösung.

Die Ausfällung der Paroteine erfolgt mit einem geeigneten Proteintrennungsmittel, wie z. B. Trichloressigsäure.

Die Farbreaktion wird mit einem einen Farbkomplex bildenden Mittel durchgeführt, wozu sich insbesondere Thiobarbitursäure eignet.

Das farbkomplexbildende Mittel sollte in ausreichender Menge im Proteinfälungsmittel löslich sein. Gemäß einer besonders bevorzugten Ausführungsform wird Trichloressigsäure verwendet, die mit Thiobarbitursäure beinahe gesättigt ist.

Vor dem Abtrennen der denaturierten Proteine wird vorzugsweise während ca. 30 Minuten bei ca. 37°C inkubiert.

Das Abtrennen der denaturierten Proteine erfolgt nach herkömmlichen Methoden, z. B. durch Zentrifugation oder Filtration.

Die photometrische Messung wird bei 443 nm durchgeführt. Bei Filtergeräten kann die Wellenlänge entsprechend variieren.

Die nachfolgenden Beispiele erläutern die Erfindung.

## Beispiel 1

0,1 ml Blutproben werden in Teströhrchen gegeben. Man fügt 1,4 ml 0,2 M (0,4 N) Oxalsäure zu und verschließt die Röhrchen. Die Röhrchen werden auf einem Heizblock bei 100°C während 5 Stunden inkubiert. Hierauf werden die Röhrchen auf Raumtemperatur abgekühlt.

Es wird 1 ml einer 0,025 M Lösung von Thiobarbitursäure in 20%iger wäßriger Trichloressigsäure zu jedem Röhrchen gegeben. Da sich die Thiobarbitursäure nicht sehr gut löst, wird bis auf 60°C erwärmt. Die erhaltene Lösung kann sofort weiterverwendet werden oder während 7 Tagen aufbewahrt werden. Die Röhrchen werden während 30 Minuten bei 37°C inkubiert und dann während 30 Minuten bei Raumtemperatur stehengelassen. Die Röhrchen werden bei 3000 UpM während 5 Minuten zentrifugiert, worauf die Überstände bei 430 nm und einer Schichtdicke von 1 cm photometrisch gemessen werden. Bei dieser Wellenlänge zeigt der durch die Thiobarbitursäure gebildete Farbkomplex einen molaren Extinktionskoeffizienten von 26.

**0 033 543**

Beispiel 2

Nach der Methode von Beispiel 1 wurden Blutproben von Diabetikern und gesunden Probanden untersucht. Hierbei wurden die folgenden $HbA_{1c}$ Werte ermittelt:

Diabetiker
| | |
|---|---|
| Patient 1 | 13,5% |
| Patient 2 | 12,9% |
| Patient 3 | 12,3% |
| Patient 4 | 10,6% |
| Patient 5 | 10,2% |
| Patient 6 | 10,0% |

gesunder Proband
| | |
|---|---|
| Proband 1 | 4,2% |
| Proband 2 | 4,4% |
| Proband 3 | 4,6% |
| Proband 4 | 4,9% |
| Proband 5 | 5,2% |
| Proband 6 | 5,3 |

Aus diesen Werten geht eindeutig hervor, daß die Diabetiker erhöhte Werte an $HbA_{1c}$ aufweisen.

Beispiel 3

Zur Herstellung des Standards werden in an sich bekannter Weise Proben, die bekannte, ansteigende Mengen von $HbA_{1c}$ enthalten, hergestellt. Nach der Methode gemäß Beispiel 1 wird der Gehalt an $HbA_{1c}$ dieser Proben bestimmt, worauf in üblicher Weise eine Standardkurve hergestellt wird.

**Patentansprüche**

1. Verfahren zur Bestimmung der Hämoglobinspezies $A_{1c}$ nach der photometrischen Methode, dadurch gekennzeichnet, daß man eine Vollblutprobe bei erhöhter Temperatur in saurer Lösung inkubiert, anschließend simultan die Proteine ausfällt und die Farbreaktion durchführt und daß man nach weiterem Inkubieren und und Abtrennen der denaturierten Proteine eine photometrische Bestimmung durchführt und den erhaltenen Wert in an sich bekannter Weise mit einem Standard vergleicht.

2. Verfahren nach Anspruch 1, dadurch gekennzeichnet, daß man die Vollblutprobe bei 95–105° C inkubiert.

3. Verfahren nach Anspruch 1, dadurch gekennzeichnet, daß man die Vollblutprobe in oxalsaurer Lösung inkubiert.

4. Verfahren nach Anspruch 3, dadurch gekennzeichnet, daß man in einer 0,4 N oxalsauren Lösung inkubiert.

5. Verfahren nach Anspruch 1, dadurch gekennzeichnet, daß man die Proteine mit Trichloressigsäure ausfällt und gleichzeitig mit Thiobarbitursäure die Farbreaktion durchgeführt.

6. Verfahren nach Anspruch 5, dadurch gekennzeichnet, daß man mit wäßriger Trichloressigsäure, die mit Thiobarbitursäure beinahe gesättigt ist, die Trichloressigsäurekonzentration der Probe auf ca. 6–10% einstellt.

7. Verfahren nach Anspruch 6, dadurch gekennzeichnet, daß die Trichloressigsäurekonzentration der Probe auf 8% eingestellt wird.

8. Verfahren nach Anspruch 1, dadurch gekennzeichnet, daß man vor dem Abtrennen der denaturierten Proteine während etwa 30 Minuten bei 37° C inkubiert.

9. Verfahren nach Anspruch 1, dadurch gekennzeichnet, daß man die denaturierten Proteine durch Zentrifugation oder Filtration abtrennt.

10. Verwendung des Verfahrens gemäß einem der Patentansprüche 1 bis 9 zur Bestimmung von Diabetes.

**Claims**

1. A method for the determination of haemoglobin species $A_{1c}$ according to the photometric method, characterized by incubating a whole blood sample at elevated temperature in acidic solution, subsequently simultaneously precipitating the proteins and carrying out the colour reaction and, after further incubation and separation of the denatured proteins, carrying out a photometric determination

3

and comparing the value abtained with a standard in a manner known per se.

2. A method according to claim 1, characterized in that the whole blood sample is incubated at 95—105°C.

3. A method according to claim 1, characterized in that the whole blood sample is incubated in oxalic acid solution.

4. A method according to claim 3, characterized in that the incubation is carried out in a 0.4 N oxalic acid solution.

5. A method according to claim 1, characterized in that the proteins are precipitated with trichloroacetic acid and the colour reaction is carried out simultaneously with thiobarbituric acid.

6. A method according to claim 5, characterized in that the trichloroacetic acid concentration of the sample is adjusted to about 6—10% with aqueous trichloroacetic acid which is almost saturated with thiobarbituric acid.

7. A method according to claim 6, characterized in that the trichloroacetic acid concentration of the sample is adjusted to 8%.

8. A method according to claim 1, characterized in that the incubation is carried out at 37°C for about 30 minutes before the separation of the denatured proteins.

9. A method according to claim 1, characterized in that the denatured proteins are separated by centrifugation or filtration.

10. The use of the method in accordance with any one of patent claims 1 to 9 for the determination of diabetes.


## Revendications

1. Procédé pour la détermination de l'hémoglobine du type $A_{1c}$ par la méthode photométrique, caractérisé en ce que l'on soumet un échantillon de sang entier à incubation à chaud en solution acide, on procède ensuite simultanément à la précipitation des protéines et à la réaction colorée et, après nouvelle incubation et séparation des protéines dénaturées, on précède à une détermination photométrique et on compare la valeur obtenue de manière connue en soi avec un étalon.

2. Procédé selon la revendication 1, caractérisé en ce que l'on soumet l'échantillon de sang entier à incubation à une température de 95 à 105°C.

3. Procédé selon la revendication 1, caractérisé en ce que l'on soumet l'échantillon de sang entier à incubation en solution contenant de l'acide oxalique.

4. Procédé selon la revendication 3, caractérisé en ce que l'on soumet à incubation dans une solution 0,4 N en acide oxalique.

5. Procédé selon la revendication 1, caractérisé en ce que l'on précipite les protéines par l'acide trichloracétique et on procède simultanément à la réaction colorée avec l'acide thiobarbiturique.

6. Procédé selon la revendication 5, caractérisé en ce que l'on règle la concentration en acide trichloracétique de l'échantillon à un niveau d'environ 6 à 10% à l'aide d'un acide trichloracétique aqueux pratiquement saturé d'acide thiobarbiturique.

7. Procédé selon la revendication 6, caractérisé ce que l'on règle la concentration en acide trichloracétique de l'échantillon à 8%.

8. Procédé selon la revendication 1, caractérisé en ce que, avant la séparation des protéines dénaturées, on soumet à incubation de 30 nm environ à 37°C.

9. Procédé selon la revendication 1, caractérisé en ce que l'on sépare les protéines dénaturées par centrifugation ou filtration.

10. Utilisation du procédé selon l'une des revendications 1 à 9 pour la détection du diabète.